# EUROPÄISCHE PATENTANMELDUNG

(11) **EP 1 693 109 A1**
(43) Veröffentlichungstag der Anmeldung: **23.08.2006**
(21) Anmeldenummer: 05003640.9
(22) Anmeldetag: 21.02.2005
(51) Int. Cl.: B01L 3/14, C12N 5/06, G01N 33/487

(54) **Behältnis zur Separation von Tumorzellen**

(71) Anmelder: HEXAL AG, 83607 Holzkirchen (DE)
(72) Erfinder: Dahm, Michael, W., Dr., Dr., 81677 München (DE)
(74) Vertreter: Secklehner, Günter

(57) **Zusammenfassung**

Die Erfindung beschreibt ein Behältnis (1) zur Separation von Tumorzellen, insbesondere disseminierten Tumorzellen (9), von einer biologischen Probe mit einem geschlossenen und einem öffenbaren Ende (2, 3). Eine thixotrope Substanz (4) ist mit einer spezifischen Dichte ausgewählt aus einem Bereich mit einer unteren Grenze von 1,055 g/cm3, vorzugsweise 1,057 g/cm3, insbesondere 1,060 g/cm3, und einer oberen Grenze von 1,070 g/cm3, vorzugsweise 1,069 g/cm3, insbesondere 1,065 g/cm3, und gegebenenfalls ein Separationsmedium (5) in Form eines Dichtegradienten mit einer spezifischen Dichte ausgewählt aus einem Bereich mit einer unteren Grenze von 1,060 g/cm3, vorzugsweise 1,065 g/cm3, insbesondere 1,070 g/cm3, und einer oberen Grenze von 1,085 g/cm3, vorzugsweise 1,080 g/cm3, insbesondere 1,075 g/cm3, enthalten.

## Beschreibung

Die Erfindung betrifft ein Behältnis zur Separation von Tumorzellen, insbesondere disseminierten Tumorzellen, von einer biologischen Probe mit einem geschlossenen und einen öf fenbaren Ende, ein Verfahren zum Nachweis von Tumorzellen, insbesondere disseminierte Tumorzellen eine Anordnung von Kompartimenten unterschiedlicher spezifischer Dichte einer biologischen Probe und zumindest eines Trennmediums zur Separation von Tumorzellen, insbesondere disseminierten Tumorzellen, in einem Behältnis mit einem geschlossenen und einem öffenbaren Ende und einen Testkit.

Nahezu alle soliden malignen Tumore haben das Potential zur Metastasenbildung. Der Prozess der Metastasierung beinhaltet die Aussaat maligner Zellen zumeist auf dem Blut- bzw. Lymphweg in entfernte Organe und die Ausbildung autonomer Tochtergeschwülste. Das Ausmaß der Filiarisierung bestimmt die Prognose eines Tumorleidens.

Der Benefit von Tumorvorsorge- oder Nachsorgeprogrammen liegt in der Früherkennung von Primärtumoren, bzw. von Rezidiven oder Metastasen noch bevor diese klinisch manifest werden. Dieses Ziel kann bislang mit den verfügbaren apparativen Techniken nicht zufriedenstellend erreicht werden.

Durch den Nachweis von disseminierten Tumorzellen, z.B. in peripherem Blut, kann frühzeitig, d.h. noch vor dem Auftreten eines klinisch erkennbaren Tumors oder einer Metastase, eine möglicherweise kurative Immunmodulations- oder Polychemotherapie eingeleitet werden. Die Quantifizierung der Tumorzellen insbesondere vor und nach einer adjuvanten Therapie stellt dabei ein wichtiges Kontrollinstrument dar.

Neben dem Nachweis und der Quantifizierung von Tumorzellen in Körperflüssigkeiten können beispielsweise auch zytologische Charakterisierungen dieser Tumorzellen unter dem Mikroskop von Interesse sein. Darüber hinaus können unter sterilen Bedingungen isolierte Tumorzellen in Kultur genommen werden, um daraus entsprechende Zelllinien zu etablieren. Zelllinien, die statt vom Primärtumor, von disseminierten zirkulierenden Tumorzellen abstammen, bieten die Möglichkeit, Prozesse der Metastasierung differenzierter untersuchen zu können. Darüber hinaus können diese Zelllinien auch zur Entwicklung von neuen und effektiveren Tumortherapeutika beitragen und helfen, neue therapeutische und diagnostische Targets zu finden.

Eine weitere interessante Möglichkeit besteht darin, vor einer Tumortherapie, die disseminierten Tumorzellen eines Krebspatienten zu gewinnen, und diese individuell auf das Ansprechen von Tumortherapeutika zu testen.

Da Körperflüssigkeiten im Allgemeinen eine Vielzahl unterschiedlicher Zellen enthalten, ist es wünschenswert, vor einer Quantifizierung bestimmter Zelltypen, wie Tumorzellen, insbesondere disseminierten Tumorzellen, diese anzureichern und gleichzeitig eine möglichst große Menge von nicht-Tumorzellen abzureichern, um die Quantifizierung zu erleichtern.

Bereits in den 60iger und 70iger Jahren wurden Tumorzellen von haematopoetischen Zellen, aufgrund ihrer unterschiedlichen Dichte, getrennt. Entsprechend dieser Daten besitzen Tumorzellen eine spezifische Dichte von < 1,040 bis >1,065 g/cm³, während Erythrozyten und polymorphonukleäre Leukozyten eine höhere Dichte aufweisen. Lymphozyten weisen hingegen eine spezifische Dichte im Bereich von 1,060 bis 1,075 g/cm³ auf und überschneiden sich somit mit der spezifischen Dichte von Tumorzellen.

Heute werden bereits unterschiedliche Methoden angewendet, um disseminierte Tumorzellen zu isolieren und zu charakterisieren. Eine Gruppe von Verfahren, wie z.B. die Filtration bzw. die Dichtegradientenzentrifugation, basieren auf den physikalischen Eigenschaften, wie Größe, bzw. Dichte der Zellen.

Eine weitere Verfahrensgruppe nutzt die spezifischen immunologischen Eigenschaften von Tumor- bzw. Blutzellen, indem die Zellen mittels spezifischer Antikörper gebunden und entweder durch positive Selektion angereichert bzw. durch negative Selektion abgereichert werden. Bei der positiven Selektion werden zur Anreicherung von epithelialen Tumorzellen, die Zellen beispielsweise mit spezifischen Antikörpern gegen epithelzell-spezifische Antigene, wie z.B. EPCAM (Epithelial Cell Adhesion Molecule, ist gleich HEA bzw. 17-1 A Antigen) und Zytokeratine, markiert und an magnetische Partikel oder fluoreszierende Moleküle gekoppelt. Die so markierten Tumorzellen werden dann mittels eines Zellseparators, wie MACS (Magnetic Cell Sorting) oder FACS (Fluorescence Activated Cell Sorting) angereichert. Die positive Selektion hat den Nachteil, dass nur Tumorzellen epithelialen Ursprungs erfasst werden können.

Diese Selektionsverfahren sind sehr aufwendig und zeitintensiv. Die Zellen werden einem großen verfahrensbedingten Stress ausgesetzt. Dadurch kann die Zellmorphologie teilweise so nachteilig verändert werden, dass eine einwandfreie zytologische Begutachtung nicht mehr möglich ist. Weiterhin können die angereicherten Zellen nicht mehr in Kultur genommen und expandiert werden.

Zusätzlich kann die individuelle Antigenpräsentation auf und in den Blut- oder Tumorzellen individuell unterschiedlich reguliert sein. Im Falle einer geringen Antigenpräsentation auf der Zelloberfläche kann dies zu schlechten Trennergebnissen führen. Weiterhin können Blut- oder Tumorzellen aber auch für die Selektion unerwünschte Oberflächenantigene exprimieren. Dies kann zu unspezifischen An- bzw. Abreicherungen und zu falsch-positiven bzw. falsch-negativen Ergebnissen führen. Weiterhin sind die Antikörper basierenden Selektion- und Nachweisverfahren in hohem Maße abhängig von der Güte der jeweiligen zum Einsatz kommenden Antikörper.

Insgesamt betrachtet wird die zelluläre, insbesondere immunzytologische Begutachtung der Zellen als hoch spezifisch angesehen, jedoch gilt sie als wenig sensibel. In jedem Falle aber sind diese Verfahren aufwendig und teuer und somit schwer für die Routinediagnostik geeignet.

Die molekulare Diagnostik, insbesondere die Amplifikationsverfahren, wie die PCR (Polymerase-Kettenreaktion), RT-PCR (Reverse Transkriptase Polymerase-Kettenreaktion) und QPCR (Quantitative Reverse Transkriptase Polymerase-Kettenreaktion) zum Nachweis tumorzellspezifischer Nukleinsäuren, wie DNA bzw. RNA, bietet eine interessante Alternative zu den zellulären Nachweisverfahren. Sämtliche auf der PCR- (Polymerase-Kettenreaktion-) Technologie aufbauenden Verfahren sind hoch sensitiv, jedoch in hohem Maße abhängig von der Güte der Nukleinsäuren in einer Probe. Nach Blutabnahme können die zellulären Nukleinsäuren, insbesondere die RNA, sehr schnell durch intra- und extrazelluläre Enzyme abgebaut werden, sodass zellspezifische, insbesondere tumorzellspezifische Nukleinsäuren nicht mehr ausreichend, bzw. überhaupt nicht mehr quantifiziert, bzw. detektiert werden können.

Zudem gilt es als erwiesen, dass Zellen und insbesondere Tumorzellen nach der Blutabnahme zellspezifische, bzw. tumorzellspezifische Genexpressionsprofile herunterfahren können. Diese Veränderungen im Genexpressionsprofil ist umso nachhaltiger, je länger die Tumorzellen in der unbearbeiteten Blutprobe verweilen. Nachdem die nachgeschalteten Nukleinsäure-Aufreinigungsverfahren, insbesondere die RNA- Extraktions-, Konzentrations- und Umschreibeverfahren, sehr anfällig sind für eine Kontamination mit exogenen Nukleasen, insbesondere mit RNasen, können trotz unübertroffener Sensitivität der molekularen Diagnostik falsch negative Ergebnisse produziert werden.

Aus dem Stand der Technik ist das OncoQuick® System zur Anreicherung disseminierter Tumorzcllcn bekannt. Die Anreicherung der zirkulierenden Tumorzellen mit OncoQuick® basiert auf einem Dichtegradienten, der speziell an die Dichte der aufzutrennenden Zellen angepasst und für 15 bis 30 ml Vollblut und Knochenmark optimiert ist. OncoQuick® besteht aus einem Zentrifugenröhrchen, das durch eine poröse Trennscheibe in zwei Kompartimente geteilt ist. Das untere Kompartiment enthält ein Trennmedium. In das obere Kompartiment können bis zu 30 ml der zu untersuchenden Probe gefüllt werden. Aufgrund der besonderen Eigenschaften des Separationsmediums und der Trennscheibe, werden die unerwünschten Blut- bzw. Knochenmarkzellen während der Zentrifugation in das untere Kompartiment sedimentiert und verdrängen dabei ein korrespondierendes Volumen des Trennmediums in das obere Kompartiment. Die Zellfraktion mit der geringeren Dichte, die u.a. Tumorzellen enthält, wird in der Interphase im oberen Kompartiment zwischen Plasma und Trennmedium angereichert und kann von dort entnommen werden.

Untersuchungen mit OncoQuick® haben nun überraschend ergeben, dass das Alter des Blutes entscheidend für den Grad und die Güte (i) der Anreicherung von disseminierten Tumorzellen und insbesondere (ii) der Abreicherung von unerwünschten Nicht-Tumorzellen ist. Bei einer Aufarbeitung von 20 ml peripherem Blut mit OncoQuick® nach 2 Stunden bzw. nach 24 Stunden, nimmt der Grad der Abreicherung von unerwünschten Nicht-Tumorzellen, verglichen mit den ursprünglich in der Blutprobe vorhandenen Blutzellen, mit einem relativen Log Abreicherungsfaktor von ca. Log 5,9 auf ca. Log 3,8 ab. Verantwortlich dafür ist eine unspezifische und zelltypabhängige Änderung, insbesondere Erhöhung der spezifischen Dichte der Blutzellen.

Aufgabe der Erfindung ist es daher ein Verfahren bzw. Komponenten zur Durchführung eines Verfahrens zur Verfügung zu stellen, mit welchem eine rasche und effiziente Anreicherung von Tumorzellen möglich ist. Teilaufgabe der Erfindung ist es nachteilige Veränderungen auf (i) zellulärer und (ii) molekularer Ebene, sowohl für das Anreicherungsverfahren selbst, als auch für die nachgeschalteten zellulären bzw. molekularen Nachweisverfahren, zu verhindern oder zumindest möglichst gering gehalten werden.

Die Aufgabe der Erfindung wird jeweils eigenständig gelöst durch (A) ein Behältnis mit einem geschlossenen und einem öffenbaren Ende, in welchem eine (a) thixotrope Substanz mit einer spezifischen Dichte, ausgewählt aus einem Bereich mit einer unteren Grenze von 1,055 g/cm³, vorzugsweise 1,057 g/cm³, insbesondere 1,060 g/cm³, und einer oberen Grenze von 1,070 g/cm³, vorzugsweise 1,069 g/cm³, insbesondere 1,065 g/cm³, und gegebenenfalls (b) ein Separationsmediums in Form eines Dichtegradienten mit einer spezifischen Dichte, ausgewählt aus einem Bereich mit einer unteren Grenze von 1,060 g/cm³, vorzugsweise 1,065 g/cm³, insbesondere 1,070 g/cm³, und einer oberen Grenze von 1,085 g/cm³, vorzugsweise 1,080 g/cm³, insbesondere 1,075 g/cm³, vorgelegt ist, (B) ein Verfahren, umfassend die Schritte a) Bereitstellung eines Behältnisses, b) die Zugabe der biologischen Proben in das Behältnis, c) Zentrifugation des Behältnisses zur Trennung der biologischen Proben in zumindest ein unteres und oberes Kompartiment, d) Durchführung molekularbiologischer, immunologischer und/oder zellulärer Diagnostik, mit den sich nach der Zentrifugation in einem Kompartiment oberhalb der thixotropen Substanz und/oder porösen Barriere befindlichen Tumorzellen der biologischen Probe, (C) eine Anordnung von Kompartimenten unterschiedlicher, spezifischer Dichte, wobei anfänglich im Bereich des geschlossenen Endes (a) ein Separationsmedium in Form eines Dichtegradienten mit einer spezifischen Dichte, ausgewählt aus einem Bereich mit einer unteren Grenze von 1,06 g/cm³, vorzugsweise 1,065 g/cm³, insbesondere 1,070 g/cm³, und einer oberen Grenze von 1,085 g/cm³, vorzugsweise 1,080 g/cm³, insbesondere 1,075 g/cm³, und gegebenenfalls anschließend (b) eine thixotrope Substanz mit einer spezifischen Dichte, ausgewählt aus einem Bereich mit einer unteren Grenze von 1,055 g/cm³, vorzugsweise 1,057 g/cm³, insbesondere 1,060 g/cm³, und einer oberen Grenze von 1,070 g/cm³, vorzugsweise 1,069 g/cm³, insbesondere 1,065 g/cm³ vorgelegt sind, wobei im Bereich des öffenbaren Endes, (c) ein Freiraum mit einem ausreichenden Volumen zur Aufnahme der biologischen Probe angeordnet ist, und nach der Zentrifugation, beginnend mit (i) dem Kompartiment im Bereich des unteren Endes des Behältnisses, Erythrozyten, anschließend (ii) ein Kompartiment aus Leukozyten, Monozyten, Lymphozyten und gegebenenfalls (iii) ein Teil des Separationsmediums, wiederum anschließend (iv) die thixotrope Substanz, darauf (v) ein Kompartiment verdünnten Separationsmediums, anschließend (vi) ein Kompartiment aus Plasma mit Thrombozyten und Tumorzellen, insbesondere disseminierten Tumorzellen, und gegebenenfalls (vii) ein Freiraum angeordnet sind, (D) einem dazu geeigneten Testkit, sowie deren (E) Verwendung.

Vorteilhaft dabei erweist sich weiters, dass optimale Trenneigenschaften der biologischen Probe erzielt werden können, wenn die Auftrennung der disseminierten Tumorzellen so rasch wie möglich nach der Probengewinnung durchgeführt werden kann. Besonders vorteilhaft ist, wenn die Verfahren der Probennahme und die Auftrennung der in der Probe enthaltenen disseminierten Tumorzellen in demselben Behältnis durchgeführt werden können.

Eine bevorzugte Ausfi.ihrungsform der Erfindung ist damit, die Bereitstellung eines evakuierbaren Behältnisses als Blutabnahmesystem, womit mit einem bereits vorhandenen Vakuum die Probenentnahme automatisch durchgeführt werden kann, und somit keine Notwendigkeit besteht, dass diejenige Person, welche die Probe entnimmt, mit der biologischen Probe in Kontakt tritt.

Eine weitere bevorzugte Ausführungsform der Erfindung ist, dass dieses Behältnis zentrifugierbar ist, wobei das Behältnis, mit welchem die biologische Probe, falls diese eine durch Punktion gewinnbare Körperflüssigkeit ist, gewonnen wird, direkt zentrifugiert werden kann und kein Zwischenschritt notwendig ist, um die biologische Probe vom Gefäß der Probenentnahme in das Gefäß zur Zentrifugation überzuführen.

Von Vorteil erweist sich des Weiteren, dass antikoagulierende und/oder aggregationshemmende Substanzen enthalten sind, wodurch einerseits die Aggregation von Thrombozyten an Tumorzellen und/oder die Körperflüssigkeit von Substanzen befreit wird, die eine Aggregation von Thrombozyten an Tumorzellen fördert und andererseits eine Gerinnung der biologischen Probe verhindert werden kann.

Die thixotrope Substanz ist aus einem Material aus einer Gruppe umfassend Kieselsäure, Bentonite, Hectorite, Kaolin, Alginate und/oder einem Gemisch daraus, ausgewählt, wobei sich durch Einwirkung mechanischer Kräfte die Substanz verflüssigt und nach Beendigung der mechanischen Beanspruchung wieder verfestigt.

Das Separationsmedium ist eine wässrige Lösung aus zumindest einem Polymer, insbesondere Sacharid und/oder Diatrizoat, wie Percoll®, Ficoll® bzw. Medien mit gleichen Trenneigenschaften, wobei dadurch ein Dichtegradient zur Verfügung steht, mit welchem eine einfache und rasche Zentrifugationsprozedur zur Separation von Zellen aufgrund ihrer unterschiedlichen Dichte durchgeführt werden kann.

In einer Weiterbildung ist vorgesehen, dass das Separationsmedium und/oder die thixotrope Substanz einen oder mehrere Farbstoffe enthält/enthalten, wodurch eine deutliche Grenze zwischen dem Separationsmedium bzw. der thixotropen Substanz und den Tumorzellen der biologischen Probe visuell sichtbar gemacht werden kann.

In einer Weiterbildung ist vorgesehen, dass eine poröse Barriere aus einer Membran, Klappe, Fritte, Sieb und/oder Filter angeordnet ist, wodurch das Behältnis in ein oberes und unteres Kompartiment unterteilt wird, wobei das Zellseparationsmedium im unteren Kompartiment vorgelegt und die Körperflüssigkeit in das obere Kompartiment eingebracht wird. Vorteilhaft dabei erweist sich, dass beim Hinzufügen der biologischen Probe es zu keiner Durchmischung mit dem Zellseparationsmedium kommt. Zugleich wird auch ein Vermischen der Kompartimente nach der Zentrifugation vermieden.

Die bevorzugte Ausführungsform ist ein evakuierbares Behältnis 1 das durch ein in das untere Drittel des Behältnisses vorgelegtes thixotropes Gel 4 in zwei Kompartimente, ein unteres 8 und ein oberes 7, unterteilt wird. Das unter das thixotrope Gel gelegte Separationsmedium 5 dient dazu, das thixotrope Gel (i) in Position zu halten und (ii) zu gewährleisten, dass während der Zentrifugation, die schwereren Zellen ungehindert durch das thixotrope Gel (bzw. das verflüssigte Gel) wandern und sich in dem ursprünglich durch das Separationsmedium gebildeten Raum ansammeln können. Wird zusätzlich eine poröse Barriere 5, (Membran, Klappe, Fritte, Sieb und/oder Filter) eingeführt, so ergeben sich folgende Möglichkeiten:

Ausführungsform 1: keine zusätzliche Vorrichtung, denn ein Vakuum kann angelegt werden.

Ausführungsform 2: zusätzlich dichtet eine thixotrope Substanz die poröse Barriere (Membran, Fritte, Sieb und/oder Filter, die Klappe) ab, damit das Vakuum besser angelegt werden kann. In diesem Falle hat die thixotrope Substanz eine geringere Dichte als das Separationsmedium und es übernimmt das Separationsmedium die Trennaufgabe des thixotropen Gels. Die poröse Barriere (Membran, Fritte, Sieb und/oder Filter, die Klappe) wird zusätzlich, zur thixotropen Substanz ohne Separationsmedium eingesetzt. In diesem Falle übernimmt die Barriere die Funktion des Separationsmediums und liegt unter der thixotropen Substanz. Die unter das thixotrope Gel gelegte poröse Barriere dient dazu, das thixotrope Gel (i) in Position zu halten und (ii) zu gewährleisten, dass während der Zentrifugation, die schwereren Zellen ungehindert durch das thixotrope Gel (bzw. das verflüssigte Gel) wandern und sich in dem ursprünglich durch die poröse Barriere gebildeten Raum ansammeln können.

Die poröse Barriere (Membran, Klappe, Fritte, Sieb und/oder Filter) wird zusätzlich zur thixotropen Substanz und Separationsmedium eingesetzt.

Die poröse Barriere weist eine Dicke, ausgewählt aus einem Bereich mit einer oberen Grenze von 15 mm, vorzugsweise 10 mm, insbesondere 5 mm, und einer unteren Grenze von 0,1 mm, vorzugsweise 1 mm, insbesondere 2 mm, auf, wodurch eine entsprechende Festigkeit vorliegt, um den Zentrifugationskräften unbeschadet Stand zu halten.

Poröse Barrieren mit einer Porengröße, ausgewählt aus einem Bereich mit einer oberen Grenze von 150 µm, vorzugsweise 100 µm, insbesondere 50 µm, und einer unteren Grenze von 1 µm, vorzugsweise 10 µm, insbesondere 20 µm, gewährleisten, dass bei der Zentrifugation Flüssigkeiten sowie korpuskuläre Bestandteile, welche eine höhere Dichte als das vorgelegte Separationsmedium, aufweisen, die Barriere ungehindert passieren können und somit das Separationsmedium während der Zentrifugation in das obere Kompartiment gedrängt wird. Die Tumorzellen kommen auf einer Ebene oberhalb der porösen Barriere zu liegen.

In einer Weiterbildung besteht die poröse Barriere aus einem hydrophoben Material und/oder ist mit einer hydrophoben Beschichtung ausgestattet, wodurch eine Anlagerung von Zellen oder anderen partikulären Bestandteilen verhindert werden kann.

Vorteilhaft erweist sich des Weiteren, dass die poröse Barriere von einem Elastomer begrenzt ist und dadurch einen dichten, insbesondere flüssigkeitsdichten Abschluss zwischen der Innenwand des Behältnisses und der Barriere selbst herstellen kann.

Durch die Anordnung eines einbringbaren Verschlusselements ist es möglich, während der Einwirkung der Zentrifugalkräfte eine Durchtrittsöffnung bereitzustellen, welche sich in Ruhe wieder schließt.

An der Innenseite kann ein Fortsatz angeordnet sein, der die poröse Barriere in ihrer Endstellung positioniert, womit ein unteres Kompartiment gebildet wird, indem die Barriere während und nach der Blutabnahme, sowie während und nach der Zentrifugation in der gewünschten Position blockiert wird.

Mit dem erfindungsgemäßen Verfahren können vorteilhafterweise eine Vielzahl unterschiedlicher, biologischer Proben, wie eine Körperflüssigkeit aus einer Gruppe, umfassend Blut und Knochenmark, Urin, Speichel, Lymphe, Exsudate, Transsudate, Spinalflüssigkeit, Samenflüssigkeit, bzw. dispergiertes Gewebe und/oder Flüssigkeiten aus natürlichen oder unnatürlichen Körperhöhlen, unabhängig vom Ursprung der biologischen Probe verwendet werden. Es müssen somit keine Adaptierungsschritte für Proben jeweils unterschiedlichen Ursprungs durchgeführt werden.

Es können Tumorzellen metastasierender, insbesondere mikrometastasierender Tumoren und/oder Neoplasien aus einer Gruppe der (1) soliden Tumore, umfassend (i) die epithelialen Tumore, wie beispielsweise Lungenkarzinome (kleinzellige und nicht-kleinzellige Lungenkarzinome), Gastrointestinaltumore (Leberzellkarzinom, Pankreaskarzinom, Ösophaguskarzinom, Magenkrebs, Dünndarmkrebs, kolorektales Karzinom), Brustkrebs, Nieren- bzw. Nebennierentumor, Blasenkrebs und Prostatakarzinom, und (ii) nicht-epithelialen Tumore, wie beispielsweise Melanom, Neuroblastom, Gehirntumor, Rhabdomyosarkom, Leiomyosarkom oder Teratokarzinom, und der (2) hämatologischen Tumore, wie beispielsweise T-Zell-Lymphoblastom, T-Zell- Leukämie, chronisch myeloische Leukämie, akute lymphatische Leukämie, chronisch lymphatische Leukämie, und/oder Lymphom, nachgewiesen werden, wodurch eine Vielzahl unterschiedlicher Tumorerkrankungen mit dem gleichen Verfahren nachgewiesen werden können.

Die biologische Probe kann verdünnt werden, wodurch bessere Separationseigenschaften erzielt werden können, indem beispielsweise bei Vorliegen der biologischen Probe in einem kleinen Volumen das Volumen vergrößert wird und/oder die Viskosität der biologischen Probe erniedrigt wird.

Von Vorteil erweist sich des Weiteren, dass das Blut in einer gerinnungshemmenden Substanz abgenommen wird, wodurch eine Koagulation des Bluts während des Verfahrens zur Separation von Tumorzellen verhindert wird.

Zudem erweist sich von Vorteil, dass der biologischen Probe zumindest eine aggregationshemmende Substanz zugefügt wird, wodurch eine Aggregation von Thrombozyten an Tumorzellen verhindert wird bzw. die biologische Probe von Substanzen befreit wird, welche eine Aggregation von Thrombozyten an Tumorzellen fördern.

Die Zentrifugation erfolgt mit einer g-Zahl, ausgewählt aus einem Bereich mit einer unteren Grenze von 500 g, vorzugsweise 800 g, insbesondere 1000 g und einer oberen Grenze von 2500 g, vorzugsweise 2000 g, insbesondere 1500 g, wodurch optimale Trenneigenschaften der disseminierten Tumorzellen von den restlichen Bestandteilen der biologischen Probe erzielt werden. Vorteilhafterweise wird zudem ohne Beschleunigung und ohne Verwendung einer Bremse zentrifugiert.

Die Zentrifugation erfolgt für eine Dauer mit einer oberen Grenze von 60 min, vorzugsweise 45 min, insbesondere 30 min, und einer unteren Grenze von 5 min, vorzugsweise 10 min, insbesondere 20 min, wobei die Separation der Tumorzellen am effizientesten erfolgt und somit die nachfolgende Gewinnung der Tumorzellen aus dem oberen Kompartiment erleichtert wird.

Die Zentrifugation erfolgt bevorzugt bei 4°C, wobei die bevorzugten Dichten (i) der thixotropen Substanz und gegebenenfalls (ii) des Separationsmediums auf diese Temperatur eingestellt sind. Bei einer Zentrifugation von 20°C, muss die Einstellung der spezifischen Dichte (i) der thixotropen Substanz und gegebenenfalls (ii) des Separationsmediums entsprechend erhöht werden.

Das Behältnis kann nach der Zentrifugation und vor Abnahme des mit Tumorzellen angereicherten Kompartiments gekühlt werden, wodurch eine klare Abgrenzung zwischen dem Kompartiment, welches keine Tumorzellen enthält und dem benachbarten die Tumorzellen enthaltenden Kompartiments erfolgt.

Die Tumorzellen können aus einem Kompartiment in und/oder unter dem Plasmakompartiment gewonnen werden, wobei durch die Anordnung der Tumorzellen in oder benachbart zum Plasmakompartiment ein optimales Milieu für die Tumorzellen geschaffen wird. Dieses Milieu bewirkt, dass einerseits die Zellen in ihrer Morphologie erhalten, sowie andererseits die Zellen vor dem Abbau von diversen Enzymen bewahrt werden, sodass deren Inhaltsstoffe, welche weiterführend analysiert werden sollen, wie z.B. DNA oder RNA, nicht durch DNAsen oder RNasen angegriffen werden.

Die dissemenierten Tumorzellen können aus dem oberen Kompartiment manuell, semiautomatisch oder automatisch entnommen werden, wodurch eine Vielzahl von unterschiedlichen Parametern aus dem oberen Kompartiment neben den disseminierten Tumorzellen bestimmt werden können, indem beispielsweise mittels eines Laborroboters Plasma automatisch gewonnen wird und serologische Routineparameter aus dem entnommenen Plasma analysiert werden.

Für die nachfolgende Diagnostik kann eine Methode aus einer Gruppe umfassend immunzytochemische Färbung, PCR (Polymerase-Kettenreaktion), RT-PCR (Reverse Transkriptase Polymerase-Kettenreaktion), QPCR (Quantitative Reverse Transkriptase Polymerase-Kettenreaktion), Zellkultur, FISH (Fluoreszenz in-situ Hybridisierung) und/oder FACS (Fluoreszenz aktivierter Zellsortierer), ausgewählt werden, wodurch sowohl molekularbiologische, immunologische und/oder zelluläre Diagnostik je nach Bedarf durchgeführt werden kann. Durch die Verwendung unterschiedlicher Nachweismethoden kann ein Ergebnis, welches mit einer ausgewählten Methode bestimmt wird, bestätigt werden. Eine Aussage über das Vorliegen eines Tumors ist für einen Patienten von großer Bedeutung und daher ist es von größerer Relevanz, dass keine falsch positiven Ergebnisse resultieren.

Im Testkit kann neben dem erfindungsgemäßen Behältnis ein Gefäß mit einem Waschpuffer, gegebenenfalls in konzentrierter Form, enthalten sein, wodurch in dem die Analyse durchführenden Labor bzw. Institut keine zusätzlichen Reagenzien benötigt werden.

Im Kit können zudem weitere Probengefäße enthalten sein, in welche die gewaschenen Tumorzellen überführt werden, und weitere Verfahrensschritte und Analysen durchgeführt werden.

Die vorliegende Erfindung wird anhand der nachfolgenden Ausführungen näher beschrieben.

Die Erfindung wird im nachfolgenden anhand des in der Zeichnung dargestellten Ausführungsbeispiels näher erläutert.

Es zeigt:

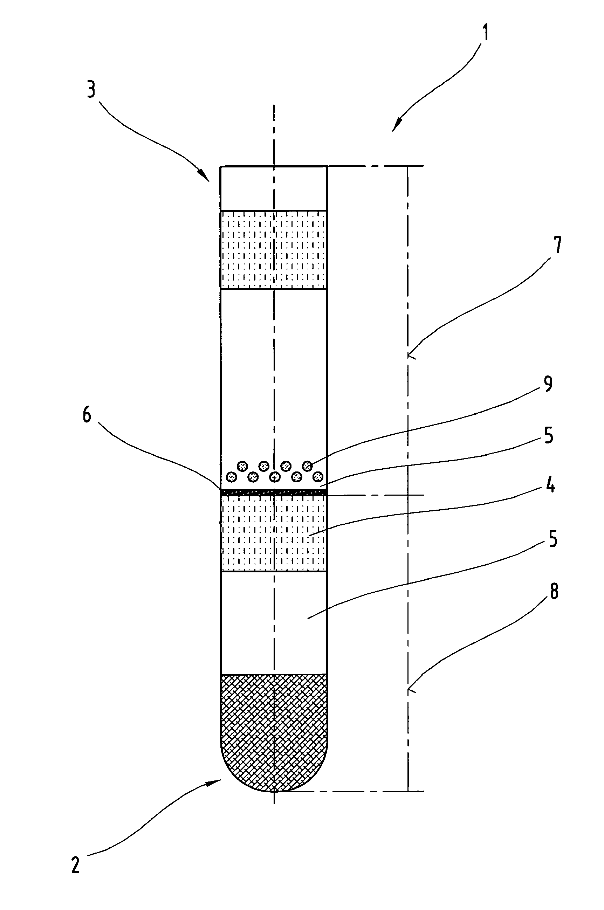

- Fig. 1: einen Längsschnitt der Anordnung der Kompartimente 7, 8 im Behältnis 1.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z. B. oben, unten, seitlich usw. auf die unmittelbar beschriebene sowie dargestellte Figur bezogen und sind bei einer Lageänderung sinngemäß auf die neue Lage zu übertragen. Des Weiteren können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Die vorliegende Erfindung betrifft ein Verfahren zur An- und/oder Abreicherung von Tumorzellen aus einer biologischen Probe, wobei eine oder mehrere Substanzen mit der biologischen Probe in einer Flüssigkeit in einem Behältnis 1 zusammengeführt und zentrifugiert wird/werden.

Das erfindungsgemäße Verfahren kann in Abhängigkeit davon, welches Kompartiment nach der Zentrifugation weiterverarbeitet wird, sowohl zur An- als auch zur Abreicherung von Tumorzellen, insbesondere disseminierten Tumorzellen 9, verwendet werden. Es wird daher nicht zwischen diesen beiden möglichen Behandlungen unterschieden, sondern allgemein von einer Anreicherung von Tumorzellen gesprochen, wobei erfindungsgemäß jedoch beide Möglichkeiten umfasst sein sollen.

Die biologische Probe kann sowohl eine Körperflüssigkeit sein als auch von einem organischen Gewebe stammen. Die Probe kann sowohl menschlichen als auch tierischen Ursprungs sein. Als Körperflüssigkeit kann Blut, Urin, Speichel, Lymphe, Exsudate, Transsudate, Spinalflüssigkeit, Samenflüssigkeit, Flüssigkeiten aus natürlichen oder unnatürlichen Körperhöhlen oder bei der Verwendung von Gewebe kann Knochenmarksgewebe oder jedes andere dispergierte bzw. homogenisierte Gewebe verwendet werden.

Das Behältnis 1 zur Gewinnung der biologischen Probe bzw. in welches die biologische Probe gefüllt wird, weist ein geschlossenes 2 und öffenbares Ende 3 auf. Im Behältnis 1 ist eine thixotrope Substanz 4 mit einer spezifischen Dichte, ausgewählt aus einem Bereich mit einer unteren Grenze von 1,055 g/cm³, vorzugsweise 1,057 g/cm³, insbesondere 1,060 g/cm³ und einer oberen Grenze von 1,070 g/cm³, vorzugsweise 1,069 g/cm³, insbesondere 1,065 g/cm³, enthalten.

In einer Weiterbildung des Behältnis 1 kann ein Separationsmedium 5 in Form eines Dichtegradienten mit einer spezifischen Dichte gleich oder höher jener der thixotropen Substanz 4, ausgewählt aus einem Bereich mit einer unteren Grenze von 1,060 g/cm³, vorzugsweise 1,065 g/cm³, insbesondere 1,070 g/cm³ und einer oberen Grenze von 1,085 g/cm³, vorzugsweise 1,080 g/cm³, insbesondere 1,075 g/cm³, zusätzlich enthalten sein.

In einer alternativen Ausführungsform kann das Separationsmedium 5 auch eine spezifische Dichte, ausgewählt aus einem Bereich mit einer unteren Grenze von 1,055 g/cm³, vorzugsweise 1,057 g/cm³, insbesondere 1,060 g/cm³ und einer oberen Grenze von 1,070 g/cm³, vorzugsweise 1,068 g/cm³, insbesondere 1,065 g/cm³ und eine poröse Barriere 6 aufweisen.

In der einfachsten Ausführungsform weist das Behältnis lediglich eine thixotrope Substanz 4, insbesondere ein thixotropes Gel, mit einer spezifischen Dichte von 1,057 bis 1,069 g/cm³, insbesondere 1,060 g/cm³, auf. Die thixotrope Substanz 4 ist im Bereich des geschlossenen Endes 2 des Behältnis 1 angeordnet.

In einer Weiterbildung der einfachsten Ausführungsform ist zusätzlich zur thixotropen Substanz 4 noch ein Separationsmedium 5 in Form eines Dichtegradienten mit einer spezifischen Dichte aus einem Bereich von 1,060 bis 1,085 g/cm³, insbesondere 1,065 bis 1,070 g/cm³, enthalten. Bei dieser Ausführungsform ist das Separationsmedium 5 im Bereich des geschlossenen Endes 2 und die thixotrope Substanz 4 oberhalb des Separationsmediums 5 im Behältnis 1 enthalten.

In einer alternativen Ausführungsform des Behältnisses 1 kann die thixotrope Substanz 4 und das Separationsmedium 5 annähernd die gleiche spezifische Dichte aus einem Bereich von 1,055 g/cm³ bis 1,070 g/cm³ aufweisen, wobei zusätzlich eine poröse Barriere 6 enthalten ist. Im Bereich des geschlossenen Endes 2 des Behältnisses 1 liegt bei dieser Ausführungsform das Separationsmedium 5, darüberliegend die poröse Barriere 6 und in Richtung des offenbaren Endes 3 des Behältnis 1 liegt die thixotrope Substanz 4 vor.

Das Separationsmedium hält die thixotrope Substanz 4, vor und während der Probenaufnahme, sowie während und nach der Zentrifugation im Behältnis 1 in Position. Zudem gewährleistet das Separationsmedium 5, dass die dichteren Komponenten der zu untersuchenden Körperflüssigkeit, bevorzugt peripheres, venöses, arterielles Blut, ein Gemisch daraus, wie Blut aus der Fingerkuppe, zentralvenöses, zentralarterielles und kordales Blut sowie Knochenmark, insbesondere Erythrozyten, Monozyten, Granulozyten und ein Teil der dichteren Lymphozyten ungehindert durch die thixotrope Substanz 4 hindurchwandern und in den durch das Separationsmedium 5 ausgebildeten Raum gelangen können.

Dabei wird das korrespondierende Volumen des Separationsmedium 5 verdrängt, womit die darüberliegende thixotrope Substanz 4, nach oben geschoben und/oder das Separationsmedium 5 durch die mit den nach unten wandernden Zellen verbrachte Flüssigkeit, vorverdünnt wird, sodass eine bestimmte Menge des verdünnten Separationsmediums 5 nach der Zentrifugation oberhalb der thixotropen Substanz 4 zu liegen kommt.

Die thixotrope Substanz 4 wird von Kieselsäure, Bentonite, Hectorite, Kaolin, Alginat und einem Gemisch daraus gebildet. Die thixotrope Substanz 4, kann in Form eines Gels vorliegen und erlaubt die Trennung des Behältnis 1 in zumindest ein unteres und zumindest ein oberes Kompartiment 7, 8 und aufgrund der spezifischen Dichte die Anreicherung von disseminierten Tumorzellen 9 im oberen 7 und die gleichzeitige Abreicherung von unerwünschten Zellen im unteren Kompartiment 8. Die thixotrope Substanz 4 erlaubt zudem die Evakuierung des oberen Kompartiments 7.

Das Separationsmedium 5, das in Form eines Dichtegradienten vorliegt, wird von einer wässrigen Lösung aus zumindest einem Polymer, insbesondere Saccharid oder Diatrizoat, bekannt unter den Handelsnamen Percoll® oder Ficoll® bzw. einer percoll- oder ficollähnlichen Substanz, gebildet. Für Behältnisse 1 aus Kunststoff wird bevorzugt Percoll® und für Behältnisse I aus Glas wird bevorzugt Ficoll® verwendet.

In einer Weiterbildung des erfindungsgemäßen Behältnis 1 kann ein Farbstoff dem Separationsmedium 5 und/oder der thixotropen Substanz 4 beigemengt werden. Als Farbstoff zur Zugabe zum Separationsmedium 5 bzw. zur thixotropen Substanz 4 kann z.B. Trypanblau verwendet werden. Durch die Zugabe wird die Unterscheidung der Grenze zwischen den einzelnen Phasen bzw. Kompartimenten unterschiedlicher Dichte erleichtert.

Das Behältnis 1 kann an seinem offenen Ende auch verschließbar sein. Das Behältnis 1 kann auch evakuiert werden. Das erfindungsgemäße Behältnis 1 kann vergleichbar mit den evakuierten Blutabnahmeröhrchen aus dem Stand der Technik ebenfalls evakuiert werden und eine thixotrope Substanz 4 bzw. ein Separationsmedium 5 und gegebenenfalls eine poröse Barriere 6 enthalten.

Wird beispielsweise als biologische Probe Blut verwendet, so kann dieses direkt mit dem erfindungsgemäßen evakuierten Behältnis 1 gewonnen werden und nach einer Zentrifugation des Behältnisses 1 die Tumorzellen 9 separiert werden.

Um mit dem evakuierten Behältnis 1 eine Trennung von disseminierten Tumorzellen 9 von den restlichen Blutbestandteilen durchführen zu können, ist das Behältnis 1 zentrifugierbar.

Die biologische Probe wird nicht notwendigerweise bereits mit dem Behältnis 1 gewonnen, sondern sie kann auch erst nach deren Gewinnung dem Behältnis 1 zugeführt werden. Wird Gewebe beispielsweise durch eine Biopsie gewonnen, kann eine Flüssigkeit, z.B. ein Puffer, zugegeben werden und die Probe wird dispergiert bzw. homogenisiert. Hierbei wird die biologische Probe erst nach der Probengewinnung dem Behältnis 1 zugeführt.

In Abhängigkeit von der Art der Körperflüssigkeit bzw. dem Gewebe wird diese/s entweder zunächst mit einem Verdünnungsmittel, bevorzugt einem Puffer, verdünnt oder direkt unverdünnt in das Behältnis 1 zugeführt.

Wird als biologische Probe Blut verwendet, so werden dem Behältnis 1 zudem antikoagulierende und/oder aggregationshemmende Substanzen zugefügt.

Um eine Agglutination des Bluts zu verhindern, können gerinnungshemmende Substanzen, wie beispielsweise EDTA oder Citrat bzw. Heparin oder CPD (Citrat, Phosphat, Dextrose) oder vergleichbare Substanzen eingesetzt werden.

Aggregationshemmende Substanzen können, z.B. mit dem als Verdünnungsmittel verwendeten Puffer, zugesetzt werden. Der bevorzugt verwendete Puffer ist Dulbecco PBS (Phosphate Buffered Saline). Als Substanzen, die eine unerwünschte Aggregation von Thrombozyten an Tumorzellen verhindern, eignen sich beispielsweise EDTA, Citrat und ACD-A (Acid Citrat Dextrose). Zusätzlich oder stattdessen können die Körperflüssigkeiten von Substanzen befreit werden, die eine Aggregation von Thrombozyten an Tumorzellen fördern. Hierbei handelt sich beispielsweise um Ionen, wie Magnesium- und Kalziumionen.

Die Dicke der porösen Barriere 6 ist aus einem Bereich mit einer oberen Grenze 15 mm, vorzugsweise 10 mm, insbesondere 5 mm und einer unteren Grenze von 0,1 mm, vorzugsweise 1 mm, insbesondere 2 mm, ausgewählt.

Die Porengröße der porösen Barriere 6 ist aus einem Bereich mit einer oberen Grenze von 150 µm, vorzugsweise 100 µm, insbesondere 50 µm und einer unteren Grenze von 1 µm, vorzugsweise 10 µm, insbesondere 20 µm ausgewählt.

Die poröse Barriere 6 kann zudem aus einem hydrophoben Material hergestellt sein und/oder mit einer hydrophoben Beschichtung ausgestattet sein.

In einer Weiterbildung der porösen Barriere 6 ist ein Elastomer angeordnet bzw. begrenzt ein Elastomer die poröse Barriere.

In einer alternativen Ausführungsform kann die poröse Barriere 6 auch von einem Stempel gebildet werden, durch den die biologische Probe durch Unterdruck in das Behältnis 1 gezogen werden kann. Dieser Stempel könnte mit einer porösen Barriere 6 ausgestattet und mit einem Elastomer, wie z.B. Gummi, umgeben sein, sodass der Stempel das Behältnis 1 dicht abschließt. Ist das Behältnis 1 durch eine bewegliche poröse Barriere 6 in Form eines Stempels ausgestattet, so kann der thixotropen Substanz 4 hierbei die Aufgabe zukommen, während der Blutabnahme, die dem offenen Ende des Behältnis 1 zugewandte Seite der porösen Barriere 6 derart abzudecken, dass im Behältnis 1 ein Vakuum angelegt werden kann. Die Zelltrennung erfolgt in dieser Ausführungsvariante durch das vorgelegte Separationsmedium 5.

Im Bereich des geschlossenen Endes 2 des Behältnisses 1 kann in einer Weiterbildung zudem eine Vorrichtung, wie z.B. ein Ring, Zapfen, Fortsatz, etc., angebracht sein, die den Stempel während und nach der Blutabnahme in einer gewünschten Position blockiert, womit ein unteres Kompartiment gebildet wird, In diesem unteren Kompartiment 1 ist das Separationsmedium 5 vorgelegt. Die Dichte des Separationsmediums 5 ist in dieser Ausführungsform aus einem Bereich von 1,055 g/cm³ bis 1,070 g/cm³, insbesondere 1,057 g/cm³ bis 1,063 g/cm³ und besonders bevorzugt 1,060 g/cm³ ausgewählt. Durch eine nachfolgende Zentrifugation, erfolgt eine Anreicherung von disseminierten Tumorzellen 9 im oberen und eine gleichzeitige Abreicherung von unerwünschten Blutzellen in das untere Kompartiment.

In einer Weiterbildung der porösen Barriere 6 kann ein einbringbares Verschlusselement angeordnet sein.

Wie bereits beschrieben, wird im erfindungsgemäßen Verfahren nach Bereitstellung des Behältnisses 1 die biologische Probe zugegeben. Die biologische Probe kann mit aggregationshemmenden und/oder gerinnungshemmenden und/oder isotonischen Lösungen verdünnt werden. Die aggregationshemmenden bzw. gerinnungshemmenden Substanzen können allerdings auch in lyophilisierter bzw. aufgesprühter Form auf der Innenseite des Behältnisses 1 und insbesondere innerhalb des oberen Kompartiments 7 angeordnet sein.

In einem weiteren Verfahrensschritt wird das Behältnis 1 zur Trennung der disseminierten Tumorzellen 9 von dem Rest der biologischen Probe zentrifugiert und in zumindest ein unteres und oberes Kompartiment 7 getrennt. Die Zentrifugation erfolgt mit einer g-Zahl, ausgewählt aus einem Bereich mit einer unteren Grenze von 500 g, vorzugsweise 800 g, insbesondere 1000 g und einer oberen Grenze von 2500 g, vorzugsweise 2000 g, insbesondere 1500 g. Die biologische Probe wird für eine Dauer mit einer oberen Grenze von 60 min, vorzugsweise 45 min, insbesondere 30 min und einer unteren Grenze von 5 min, vorzugsweise 10 min, insbesondere 20 min, zentrifugiert.

Die Zentrifugation erfolgt bevorzugt bei + 4 °C; kann alternativ aber auch bei Raumtemperatur durchgeführt werden. Die Zentrifugation erfolgt bevorzugt bei 4 °C, wobei die bevorzugte Dichten (i) der thixotropen Substanz und gegebenenfalls (ii) des Separationsmediums auf diese Temperatur eingestellt sind. Da die Dichte bei steigenden Temperaturen abnimmt, muss bei einer Zentrifugation von 20 °C die Einstellung der spezifischen Dichte (i) der thixotropen Substanz und gegebenenfalls (ii) des Separationsmediums entsprechend erhöht werden.

Nach der Zentrifugation und vor der Abnahme des mit Tumorzellen angereicherten Kompartiments kann das Behältnis 1 zusätzlich gekühlt werden. Durch die Kühlung, insbesondere kurzzeitige starke Abkühlung, können die in dem unteren Kompartiment 8 des Behältnisses 1 befindlichen Erythrozyten und Leukozyten immobilisiert werden. Die Abkühlung kann in flüssigem Stickstoff erfolgen. Durch die Abkühlung wird ein Vermischen der Zellen aus den verschiedenen Kompartimenten verhindert, wodurch falsch positive Testergebnisse ausgeschlossen werden können.

Die disseminierten Tumorzellen 9 werden aus einem Kompartiment 7 oberhalb der thixotropen Substanz 4, bzw. oberhalb der porösen Barriere 6 gewonnen. Im obersten Kompartiment liegt Plasma, ein Plasma/PBS bzw. ein Plasma/Puffergemisch, das die Proteine des Plasmas enthält, vor.

Plasma hält Zellen für die Dauer des Transports zum Nachweislabor vital. Nach einer Auf reinigung mit dem erfindungsgemäßen Verfahren, z.B. noch auf der Krankenstation oder beim niedergelassenen Arzt, liegt die angereicherte und die disseminierten Tumorzellen 9 enthaltende Zellfraktion in einem solchen oberen Kompartiment 7, d.h. in einem großen Volumen von Plasma vor. Plasma bietet einerseits eine physiologische Umgebung und bewahrt daher die Zellmorphologie sowie die Funktionalität der Zellen. Dadurch wird beispielsweise die biologische Probe von degradierenden Enzymaktivitäten bewahrt und insbesondere die zelluläre RNA vor dem Abbau durch RNasen, geschützt.

Die Gewinnung der Tumorzellen erfolgt entweder (i) manuell, d.h. das Behältnis 1 wird geöffnet, und das obere Kompartiment 7 wird durch Dekantieren oder Pipettieren in ein neues Gefäß überführt (ii) semiautomatisch, wie z.B. das Überführen in eine evakuierte Probenkartusche mit Zell- bzw. Nukleinsäure-konservierenden Eigenschaften, wie dies in der WO 03/09131 beschrieben ist oder (iii) automatisch, wie z.B. durch einen Pipettierroboter. Zusätzlich können Wasch- und/oder Konservierungsschritte mit der Flüssigkeit des oberen Kompartiments 7 durchgeführt werden. Es können sowohl nur die Tumorzellen 9 als auch das gesamte obere Kompartiment 7 entnommen werden.

Zudem können aus dem oberen Kompartiment 7 auch Routineparameter der Serologie, wie dies in jedem standardisierten Labor durchgeführt werden kann, bestimmt werden.

Im Anschluss an die Trennung der Tumorzellen von der restlichen biologischen Probe, wird die Diagnostik durchgeführt. Es kann sowohl eine Diagnostik auf molekularbiologischer, immunologischer und/oder zellulärer Basis durchgeführt werden. Als Nachweismethoden seien immunzytochemische Färbung, Polymerase Kettenreaktion, reverse Transkriptase Polymerase Kettenreaktion, Zellkultur, Fluoreszenz in-situ Hybridisierung und/oder Fluoreszenz aktivierte Zellensortierung genannt. Die Aufzählung der im Anschluss an die Gewinnung der disseminierten Tumorzellen 9 angeführten Methoden ist nur eine exemplarische Aufstellung und erhebt bei weitem keinen Anspruch auf Vollständigkeit.

Mit dem erfindungsgemäßen Behältnis 1 und dem erfindungsgemäßen Verfahren kann die biologische Probe innerhalb kürzester Zeit aufgearbeitet werden. Optimale Trenneigenschaften werden erzielt, wenn die Auftrennung der disseminierten Tumorzellen 9 zumindest am gleichen Tag der Probengewinnung, wie z.B. am Tag der Blutabnahme, erfolgt. Ist das Blut älter als 1 Tag, verbleibt nach der Zentrifugation ein höherer Anteil unerwünschter Blutzellen im oberen Kompartiment 7, welches auch die angereicherten disseminierten Tumorzellen 9 enthält. Dies hat zur Folge, dass eine höhere Zell- bzw. damit eine höhere Gesamt- Nukleinsäuremenge angereichert wird und es somit zu einer Überlastung der zellulären bzw. molekularen Nachweismethoden kommen kann. Es werden beispielsweise mehr Objektträger für immunzytologische Nachweismethoden benötigt. Um ein Überladen der reverse Transkriptase Polymerase-Kettenreaktion (RT-PCR) oder der quantitativen Polymerase- Kettenreaktion (QPCR) durch höhere Nukleinsäuremengen auszuschließen, können nur Teilmengen der biologischen Probe eingesetzt werden. Dies kann wiederum zu einer Verminderung der diagnostischen Aussagefähigkeit führen, weil nur ein Anteil der ursprünglich gewonnenen biologischen Probe eingesetzt wird.

Bei Blut, welches älter als 1 Tag ist, ist zudem die Wahrscheinlichkeit erhöht, dass Tumormarker positive Nichttumorzellen mitangereichert werden und somit mit falsch positiven Ergebnissen zu rechnen ist. Zudem wird in älteren, biologischen Proben die zelluläre RNA sehr schnell abgebaut, sodass die Gefahr besteht, dass falsch negative Ergebnisse produziert werden.. Das erfindungsgemäße Verfahren ermöglicht hingegen eine sofortige Aufbereitung der biologischen Probe beim niedergelassenen Arzt oder in einer Patientenstation einer Klinik und damit eine optimale Ausgangsposition für die nachgeschaltete zelluläre bzw. molekulare Diagnostik.

Nach Durchführung der Zentrifugation liegen zumindest zwei Kompartimente, ein oberes 7 und ein unteres 8 Kompartiment vor. Die beiden Kompartimente sind wiederum von Kompartimenten unterschiedlicher spezifischer Dichte unterteilt. In der Fig. 1 wird die Anordnung der einzelnen Kompartimente gezeigt. Beginnend mit dem Kompartiment im Bereich des geschlossenen Endes 2 des Behältnisses 1 befinden sich (i) Erythrozyten, anschließend liegt (ii) ein Kompartiment mit Leukozyten, Monozyten, Lymphozyten, und gegebenenfalls (iii) ein Teil des Separationsmediums 5, wiederum anschließend (iv) die thixotrope Substanz 4, und gegebenenfalls die poröse Barriere 6, darauffolgend (v) ein Kompartiment des Separationsmediums 5, welches mit Flüssigkeit von nach unten wandernden Zellen verdünnt wurde, und darauffolgend (vi) ein Kompartiment, beinhaltend Plasma mit Thrombozyten und an der unteren Grenze des Kompartiments die disseminierten Tumorzellen 9, und gegebenenfalls (vii) ein Freiraum vor.

Das Behältnis 1 kann in einem Testkit enthalten sein. Im Testkit kann zudem ein Behältnis 1 mit einem Waschpuffer, gegebenenfalls in konzentrierter Form in Lösung oder in pulverisierter Form, zur Verwendung in einer Verdünnung mit einem Lösungsmittel enthalten sein.

Der Testkit kann des Weiteren weitere Probengefäße enthalten, in welche, nach erfolgter Zentrifugation, (i) die disseminierten Tumorzellen 9, bzw. in einer bevorzugten Anwendungsform (ii) das gesamte obere, die disseminierten Tumorzellen 9 enthaltende Kompartiment 7, übergeführt werden können. In diesen Gefäßen können die notwendigen Wasch-, Konzentrierungs- und Konservierungsschritte an den angereicherten Tumorzellen 9 vollzogen werden, sodass die Zellen oder die aus den Zellen aufgeschlossenen Nukleinsäuren und dann einer weiterführenden zellulären oder molekularen Diagnostik zugeführt werden können.

Die spezifischen Dichten der oben angeführten Kompartimente gliedern sich wie folgend von dem geschlossenen Ende 2 des Behältnisses 1 beginnend:

| Kompartiment mit | spezifische Dichte (g/cm³) |
|---|---|
| Erythrozyten | 1,092 bis 1,097 |
| Leukozyten | 1,075 bis 1,085 |
| Lymphozyten/Monozyten | 1,060 bis 1,075 |
| Separationsmedium | 1,070 bis 1,085 |
| thixotrope Substanz | 1,055 bis 1,070 |
| Separationsmedium* | <1,055 bis <1,070 |
| Tumorzellen, disseminiert | 1,040 bis 1,70 |
| Plasma und Thrombozyten | 1,034 bis 1,040 |

| | |
|---|---|
| * das Separationsmedium wird mit der von nach unten wandernden, Zellen transportierenden Flüssigkeit verdünnt. | |

Die disseminierten Tumorzellen 9 können zum Nachweis von metastasierenden, insbesondere mikrometastasierenden Tumoren und/oder Neoplasien aus einer Gruppe der (1) soliden Tumore, umfassend (i) die epithelialen Tumore, wie beispielsweise Lungenkarzinome (kleinzellige und nicht-kleinzellige Lungenkarzinome, Gastrointestinaltumore (Leberzellkarzinom, Pankreaskarzinom, Ösophaguskarzinom, Magenkrebs, Dünndarmkrebs, kolorektales Karzinom), Brustkrebs, Nieren- bzw. Nebennierentumor, Blasenkrebs und Prostatakarzinom, und (ii) nicht-epithelialen Tumore, wie beispielsweise Melanom, Neuroblastom, Gehirntumor, Rhabdomyosarkom, Leiomyosarkom oder Teratokarzinom, und der (2) hämatologischen Tumore, wie beispielsweise T-Zell-Lymphoblastom, T-Zell- Leukämie, chronisch myeloische Leukämie, akute lymphatische Leukämie, chronisch lymphatische Leukämie und/oder Lymphom, verwendet werden.

Nachdem in der gesammelten Tumorzellfraktion auch Thrombozyten angereichert werden, kann es für die zelluläre Diagnostik vorteilhaft sein, dass, wenn die gesammelten Zellen z.B. auf einem Objektträger aufgebracht werden sollen die Thrombozyten noch zumindest einmal mit einem Puffer (z.B. PBS, bzw. PBS mit 0,1 % bis 7 % BSA) gewaschen und durch Zentrifugation bei ca. 200 g für 10 min von den Zellen abgetrennt werden.

### Ausführungsbeispiel:

In einem Blutgefäß ist eine thixotrope Substanz 4 mit einer spezifischen Dichte von 1,060 g/cm³ und ein Separationsmedium 5, Percoll, mit einer spezifischen Dichte von 1,070 g/cm³, enthalten. Mit aus dem Stand der Technik bekannten Methoden wird aus einer Vene mit einem Nadelhalter Blut in das Behältnis 1 abgenommen. Im Behältnis 1 ist zudem eine aggregationshemmende, gerinnungshemmende (Heparin) Substanz enthalten. Bei langsamer Beschleunigung und ohne Bremse wird für 20 min bei 1000 g und 4 °C zentrifugiert. Nach der Zentrifugation wird das gesamte Kompartiment oberhalb der thixotropen Substanz 4 abgenommen und einer weiterführenden Analyse zugeführt.

Die Figur zeigt eine mögliche Ausführungsvariante des Behältnis 1, insbesondere der Anordnung der Kompartimente im Behältnis, wobei an dieser Stelle bemerkt sei, dass die Erfindung nicht auf die speziell dargestellte Ausführungsvariante derselben eingeschränkt ist, sondern vielmehr auch diverse Kombinationen möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvariante möglich sind, vom Schutzumfang mitumfasst.

Der Ordnung halber sei abschließend darauf hingewiesen, dass zum besseren Verständnis des Aufbaus des Behältnis 1 dieses bzw. deren Bestandteile teilweise unmaßstäblich und/oder vergrößert und/oder verkleinert dargestellt wurden.

Die den eigenständigen erfinderischen Lösungen zugrundeliegende Aufgabe kann der Beschreibung entnommen werden.

### Bezugszeichenaufstellung

- 1: Behältnis
- 2: geschlossene Ende
- 3: öffenbare Ende
- 4: thixotrope Substanz
- 5: Seperationsmedium

- 6: poröse Barriere
- 7: obere Kompartiment
- 8: untere Kompartiment
- 9: disseminierte Tumorzelle

## Patentansprüche

1. Behältnis (1) zur Separation von Tumorzellen, insbesondere disseminierten Tumorzellen (9), von einer biologischen Probe mit einem geschlossenen und einem öffenbaren Ende (2, 3), **dadurch gekennzeichnet, dass** eine thixotrope Substanz (4) mit einer spezifischen Dichte ausgewählt aus einem Bereich mit einer unteren Grenze von 1,055 g/cm³, vorzugsweise 1,057 g/cm³, insbesondere 1,060 g/cm³, und einer oberen Grenze von 1,070 g/cm³, vorzugsweise 1,069 g/cm³, insbesondere 1,065 g/cm³, und gegebenenfalls ein Separationsmedium (5) in Form eines Dichtegradienten mit einer spezifischen Dichte ausgewählt aus einem Bereich mit einer unteren Grenze von 1,060 g/cm³, vorzugsweise 1,065 g/cm³, insbesondere 1,070 g/cm³, und einer oberen Grenze von 1,085 g/cm³, vorzugsweise 1,080 g/cm³, insbesondere 1,075 g/cm³, enthalten ist.

2. Behältnis (1) nach Anspruch 1, **dadurch gekennzeichnet, dass** das Behältnis (1) evakuierbar ist.

3. Behältnis (1) nach Anspruch 1 oder 2, **dadurch gekennzeichnet, dass** das Behältnis (1) zentrifugierbar ist.

4. Behältnis (1) nach einem der Ansprüche 1 bis 3, **dadurch gekennzeichnet, dass** zumindest eine antikoagulierende und/oder aggregationshemmende Substanz enthalten ist.

5. Behältnis (1) nach einem der Ansprüche 1 bis 4, **dadurch gekennzeichnet, dass** die thixotrope Substanz (4) aus einem Material aus einer Gruppe umfassend Kieselsäure, Bentonite, Hectorite, Kaolin, Alginate und/oder einem Gemisch daraus, ausgewählt ist.

6. Behältnis (1) nach einem der Ansprüche 1 bis 5, **dadurch gekennzeichnet, dass** das Separationsmedium (5) eine wässrige Lösung aus zumindest einem Polymer, insbesondere auf Silikatbasis und/oder einem hochmolekularen Kohlenhydrat, insbesondere Saccharid, Diatrizoat, wie z.B. Percoll®, Ficoll® bzw. Medien mit gleichen Trenneigenschaften, ist.

7. Behältnis (1) nach einem der Ansprüche 1 bis 6, **dadurch gekennzeichnet, dass** das Separationsmedium (5) und/oder die thixotrope Substanz (4) einen oder mehrere Farbstoffe enthält.

8. Behältnis (1) nach einem der Ansprüche 1 bis 7, **dadurch gekennzeichnet, dass** eine poröse Barriere (6) angeordnet ist.

9. Behältnis (1) nach Anspruch 8, **dadurch gekennzeichnet, dass** die poröse Barriere (6) von einer Membran, Klappe, Fritte, Sieb und/oder Filter gebildet ist.

10. Behältnis (1) nach Anspruch 8 oder 9, **dadurch gekennzeichnet, dass** die poröse Barriere (6) bewegbar ist.

11. Behältnis (1) nach einem der Ansprüche 8 bis 10, **dadurch gekennzeichnet, dass** die poröse Barriere (6) eine Dicke ausgewählt aus einem Bereich mit einer oberen Grenze von 15 mm, vorzugsweise 10 mm, insbesondere 5 mm, und einer unteren Grenze von 0,1 mm, vorzugsweise 1 mm, insbesondere 2 mm, aufweist.

12. Behältnis (1) nach einem der Ansprüche 8 bis 11, **dadurch gekennzeichnet, dass** die poröse Barriere (6) eine Porengröße ausgewählt aus einem Bereich mit einer oberen Grenze von 150 µm, vorzugsweise 100 µm, insbesondere 50 µm, und einer unteren Grenze von 1 µm, vorzugsweise 10 µm, insbesondere 20 µm, aufweist.

13. Behältnis (1) nach einem der Ansprüche 8 bis 12, **dadurch gekennzeichnet, dass** die poröse Barriere (6) aus einem hydrophoben Material besteht und/oder mit einer hydrophoben Beschichtung ausgestattet ist.

14. Behältnis (1) nach einem der Ansprüche 8 bis 13, **dadurch gekennzeichnet, dass** die poröse Barriere (6) von einem Elastomer begrenzt ist.

15. Behältnis (1) nach einem der Ansprüche 8 bis 14, **dadurch gekennzeichnet, dass** die poröse Barriere (6) mit einem einbringbaren Verschlusselement versehen ist.

16. Behältnis (1) nach einem der Ansprüche 8 bis 15, **dadurch gekennzeichnet, dass** an der Innenseite ein Fortsatz angeordnet ist, der die poröse Barriere (6) in ihrer Endstellung positioniert.

17. Verwendung des Behältnis (1) nach einem der Ansprüche 1 bis 16 zur Separation von Tumorzellen, insbesondere disseminierten Tumorzellen (9), von einer biologischen Probe.

18. Verfahren zum Nachweis von Tumorzellen, insbesondere disseminierten Tumorzellen (9), umfassend die Schritte: (a) Bereitstellung eines Behältnisses (1) nach einem der Ansprüche 1 bis 16, (b) Zugabe der biologischen Probe in das Behältnis (1), (c) Zentrifugation des Behältnisses (1) zur Trennung der biologischen Probe in zumindest ein unteres und oberes Kompartiment (7, 8), (d) Durchführung molekularbiologischer, immunologischer und/oder zellulärer Diagnostik mit den sich nach der Zentrifugation in einem Kompartiment oberhalb der thixotropen Substanz und/oder der porösen Barriere (6) befindlichen Tumorzellen der biologischen Probe.

19. Verfahren nach Anspruch 19, **dadurch gekennzeichnet, dass** als biologische Probe eine Körperflüssigkeit aus einer Gruppe umfassend Blut und Knochenmark, Urin, Speichel, Lymphe, Exsudate, Transsudate, Spinalflüssigkeit, Samenflüssigkeit, bzw. dispergiertes Gewebe und/oder Flüssigkeiten aus natürlichen oder unnatürlichen Körperhöhlen, verwendet wird.

20. Verfahren nach Anspruch 18 oder 19, **dadurch gekennzeichnet, dass** Tumorzellen metastasierender, insbesondere mikrometastasierender Tumoren und/oder Neoplasien aus einer Gruppe der (1) soliden Tumore, umfassend (i) die epithelialen Tumore, wie beispielsweise Lungenkarzinome (kleinzellige und nicht-kleinzellige Lungenkarzinome), Gastrointestinaltumore (Leberzellkarzinom, Pankreaskarzinom, Ösophaguskarzinom, Magenkrebs, Dünndarmkrebs, kolorektales Karzinom), Brustkrebs, Nieren- bzw. Nebennierentumor, Blasenkrebs und Prostatakarzinom, und (ii) nicht-epithelialen Tumore, wie beispielsweise Melanom, Neuroblastom, Gehirntumor, Rhabdomyosarkom, Leiomyosarkom oder Teratokarzinom, und der (2) hämatologischen Tumore, wie beispielsweise T-Zell-Lymphoblastom, T-Zell- Leukämie, chronisch myeloische Leukämie, akute lymphatische Leukämie, chronisch lymphatische Leukämie, und/oder Lymphom, nachgewiesen werden.

21. Verfahren nach einem der Ansprüche 18 bis 20, **dadurch gekennzeichnet, dass** die biologische Probe verdünnt wird.

22. Verfahren nach einem der Ansprüche 18 bis 21, **dadurch gekennzeichnet, dass** das Blut in einer gerinnungshemmenden Substanz abgenommen wird.

23. Verfahren nach einem der Ansprüche 18 bis 22, **dadurch gekennzeichnet, dass** der biologischen Probe zumindest eine aggregationshemmende Substanz zur Vermeidung der Aggregation von Thrombozyten an Tumorzellen zugegeben wird.

24. Verfahren nach einem der Ansprüche 18 bis 23, **dadurch gekennzeichnet, dass** die Zentrifugation mit einer g-Zahl ausgewählt aus einem Bereich mit einer unteren Grenze von 500 g, vorzugsweise 800 g, insbesondere 1000 g, und einer oberen Grenze von 2500 g, vorzugsweise 2000 g, insbesondere 1500 g, durchgeführt wird.

25. Verfahren nach einem der Ansprüche 18 bis 24, **dadurch gekennzeichnet, dass** die biologische Probe für eine Dauer mit einer oberen Grenze von 60 Minuten, vorzugsweise 45 Minuten, insbesondere 30 Minuten, und einer unteren Grenze von 5 Minuten, vorzugsweise 10 Minuten, insbesondere 20 Minuten, zentrifugiert wird.

26. Verfahren nach einem der Ansprüche 18 bis 25, **dadurch gekennzeichnet, Dass** das Behältnis gekühlt, vorzugsweise bei 4 °C, wobei die bevorzugten Dichten (i) der thixotropen Substanz und gegebenenfalls (ii) des Separationsmediums auf diese Temperatur eingestellt sind, wird.

27. Verfahren nach einem der Ansprüche 18 bis 26, **dadurch gekennzeichnet, dass** das Behältnis (1) nach der Zentrifugation und vor der Abnahme des mit Tumorzellen angereicherten Kompartiments gekühlt wird,

28. Verfahren nach einem der Ansprüche 18 bis 26, **dadurch gekennzeichnet, dass** die Tumorzellen aus dem Kompartiment in und/oder unter dem Plasmakompartiment gewonnen werden.

29. Verfahren nach einem der Ansprüche 18 bis 28, **dadurch gekennzeichnet, dass** die disseminierten Tumorzellen (9) aus einem oberen Kompartiment (7) manuell, semiautomatisch und/oder automatisch entnommen werden.

30. Verfahren nach einem der Ansprüche 18 bis 29, **dadurch gekennzeichnet, dass** Routineparameter der Serologie aus dem obersten Kompartiment, welches Plasma ist, bestimmt werden.

31. Verfahren nach einem der Ansprüche 18 bis 30, **dadurch gekennzeichnet, dass** zur Diagnostik zumindest eine Methode aus einer Gruppe umfassend Immuncytochemische Färbung, PCR (Polymerase-Kettenreaktion), RT-PCR (Reverse Transkriptase Polymerase-Kettenreaktion), Zellkultur, FISH (Fluoreszenz in-situ Hybridisierung) und/oder FACS (Fluoreszenz aktivierter Zellsortierer), ausgewählt wird.

32. Anordnung von Kompartimenten unterschiedlicher spezifischer Dichte einer biologischen Probe und zumindest eines Trennmediums zur Separation von Tumorzellen, insbesondere disseminierten Tumorzellen (9), in einem Behältnis (1) mit einem geschlossenen und einem öffenbaren Ende (2, 3), **dadurch gekennzeichnet, dass** anfänglich im Bereich des geschlossenen Endes (2) ein Separationsmedium (5) in Form eines Dichtegradienten mit einer spezifischen Dichte ausgewählt aus einem Bereich mit einer unteren Grenze von 1,060 g/cm³, vorzugsweise 1,065 g/cm³, insbesondere 1,070 g/cm³, und einer oberen Grenze von 1,085 g/cm³, vorzugsweise 1,080 g/cm³, insbesondere 1,075 g/cm³, und gegebenenfalls anschließend eine thixotrope Substanz (4) mit einer spezifischen Dichte ausgewählt aus einem Bereich mit einer unteren Grenze von 1,055 g/cm, vorzugsweise 1,057 g/cm³ insbesondere 1,060 g/cm³, und einer oberen Grenze von 1,070 g/cm³, vorzugsweise 1,069 g/cm³, insbesondere 1,065 g/cm³, wobei im Bereich des öffenbaren Endes (3) ein Freiraum mit einem ausreichenden Volumen zur Aufnahme der biologischen Probe angeordnet ist und nach der Zentrifugation (i) beginnend mit dem Kompartiment im Bereich des unteren Endes (2) des Behältnis (1) Erythrozyten, (ii) anschließend ein Kompartiment aus Leukozyten, Lymphozyten, Monozyten und gegebenenfalls (iii) ein Teil des Separationsmediums (5), wiederum anschließend (iv) die thixotrope Substanz (4), darauf (v) ein Kompartiment verdünnten Separationsmediums (5), anschließend (vi) ein Kompartiment aus Plasma mit Thrombozyten und Tumorzellen, insbesondere disseminierten Tumorzellen (9), und gegebenenfalls (vii) ein Freiraum angeordnet sind.

33. Testkit, **dadurch gekennzeichnet, dass** ein Behältnis (1) nach einem der Ansprüche 1 bis 16, enthalten ist.

34. Testkit nach Anspruch 33, **dadurch gekennzeichnet, dass** zumindest ein Behältnis (1) mit einem Waschpuffer, gegebenenfalls in konzentrierter Form, enthalten ist.

35. Testkit nach Anspruch 33 oder 34, **dadurch gekennzeichnet, dass** weitere Probengefäße enthalten sind.
